# EUROPÄISCHE PATENTSCHRIFT

(11) **EP 0 708 180 B1**
(45) Veröffentlichungstag und Bekanntmachung des Hinweises auf die Patenterteilung: **23.06.2004**
(21) Anmeldenummer: 95116244.5
(22) Anmeldetag: 16.10.1995
(51) Int. Cl.: C12N 15/31, C12N 15/67

(54) **Verbessertes, induktionsfreies Verfahren zur Gentechnischen Herstellung von Glutarylamidase**
Improved, inductionless process for the genetical preparation of glutarylamidase
Procédé amelioré, sans inducteur, pour la préparation par recombinaison génétique de la glutarylamidase

(30) Priorität: 19.10.1994 DE 4437420
(43) Veröffentlichungstag der Anmeldung: 24.04.1996
(73) Patentinhaber: BIOCHEMIE Gesellschaft m.b.H., 6250 Kundl Tirol (AT)
(72) Erfinder: Aretz, Werner, Dr., D-61462 Königstein (DE); Holst, Ulrich, Dr., D-65582 Diez (DE); Koller, Klaus-Peter, Dr., D-65812 Bad-Soden (DE)
(74) Vertreter: Grubb, Philip William

(56) Entgegenhaltungen:
- EP-A- 0 469 919
- EP-A- 0 504 798
- BIOTECHNOLOGY, Bd. 9, Nr. 2, 1991, Seiten 188-191, XP002010439 T. ISOGAI ET AL.: "Construction of a 7-ACA biosynthetic operon and direct production of 7ACA in A. chrysogenum"

## Beschreibung

Bei der enzymatischen Herstellung von 7-Amino-Cephalosporansäure aus Cephalosporin C wird für die Abspaltung der Glutarylseitenkette das Enzym Glutarylamidase (GA) essentiell benötigt. In der europäischen Patentanmeldung EP-A-0 469 919 wird nun ein gentechnisches Verfahren zur Herstellung von GA beschrieben, bei dem die Expression von GA über den Tac-Promotor induziert wird. Die Ausbeuten an GA sind nur gering. In der EP-A-0 504 798 wurde nun ein Verfahren beschrieben, mit dem unter Verwendung von transformierten E.coli Bakterien und einem speziell abgestimmten Fermentationsverfahrens hohe Ausbeuten an GA (7000 bis 10000 U/I Kulturmedium) gelingen. Der Einsatz dieses Verfahrens macht das Gesamtverfahren erst wirtschaftlich.

Es wurde nun gefunden, daß durch die konstitutive Expression des GA-Gens, insbesondere durch die Inaktivierung des ebenfalls plasmid-codierten Lac I^{q}-Repressors und/oder durch die Verringerung der Expression des intracellulär aktiven Selektionsmarkers, insbesondere des plasmid-codierten Selektionsmarkers Chloramphenicol-Acetyltransferase (CAT), eine weitere überraschende Verbesserung der Ausbeute auf ca. das zwei- bis zweieinhalbfache erzielt werden kann.

Gegenstand der vorliegenden Erfindung ist daher ein Verfahren zur Herstellung von GA in Bakterien, wobei das GA-Gen konstitutiv von einem Expressionsvektor exprimiert wird. Vorzugsweise ist der Promotor zur konstitutiven Expression des GA-Gens ein Trc- oder Trc-äquivalenter Promotor, der keinen oder einen nicht-funktionellen Repressor besitzt.

Insbesondere ist es vorteilhaft, wenn der Expressionsvektor zur konstitutiven Expression des GA-Gens zusätzlich ein Selektionsgen enhält, das für ein in Bakterien bleibendes, nicht periplasmatisches Protein, beispielsweise die Chloramphenicolacetyltransferase (CAT), kodiert, vor allem wenn dieses Selektionsprotein nur schwach exprimiert wird.

Die Inaktivierung des Lac I^{q}-Repressor-Gens resultiert in einer starken, kontinuierlichen Expression und Sekretion des GA-Enzyms, ohne daß Wachstumsnachteile für den E.coli Stamm entstehen oder Zell-Lyse infolge der Überexpression auftritt.

Es ist daher auch denkbar, andere konstitutive, d. h. nicht über Induktion geregelte Promotoren für die Expression der GA zu verwenden. Im Gegensatz zu den E.coli Klonen T 363 brauchen entsprechende rekombinante Klone nicht mehr mit IPTG induziert werden, was zu einer weiteren Vereinfachung des Verfahrens führt.

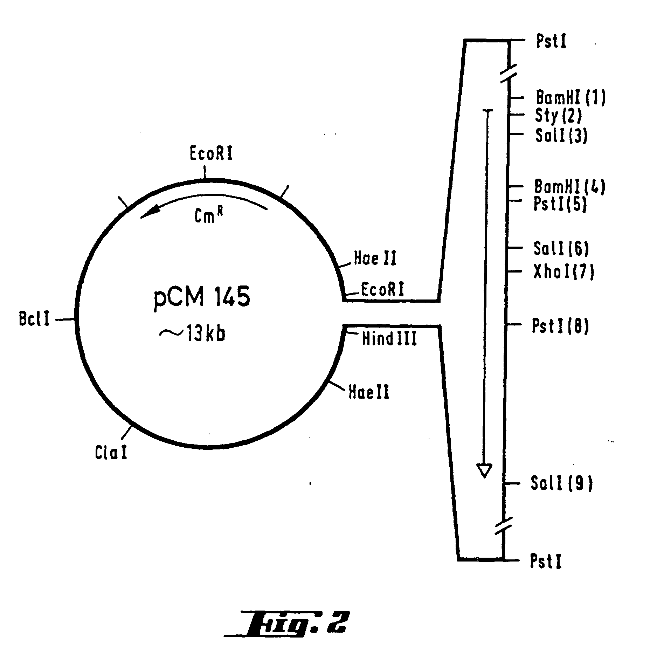

In der EP-A-0 504 798 ist die Konstruktion des Expressionsplasmides T 363 sowie die Transformation in E.coli K 12 W 3110 dargestellt. Ferner wird das Plasmid pCM 145 beschrieben, das bereits bei der Deutschen Sammlung für Mikroorganismen unter der Hinterlegungs-Nr. DSM 6409 gemäß Budapester Vertrag hinterlegt wurde und u. a. den Replikationsursprung des bekannten "low copy" Plasmids pACYC 184 (A. C. Y. Chang u. S. N. Cohen, J. Bad. 134 (1978) 1141 bis 1156) sowie das komplette Gen für eine GA aus Pseudomonas enthält. Fig. 2 zeigt eine Restriktionskarte des Plasmids pCM 145 mit Numerierung der für Klonierungen wichtigen Restriktionsenzymschnittstellen.

Zur Einklonierung des GA-Gens in den Vektor pTrc 99 A (E. Amann et al., Gene 69 (1988) 301 bis 315) wird ein synthetischer Linker (SEQ ID NO: 1 und SEQ ID NO: 2) benötigt:

(NcoI)                                                                                    (StyI)
C ATG CTG AGA GTT CTG CAC CGG GCG GCG TCC GC (SEQ ID NO: 1)
CAA GGC GGA CGC CGC CCG GTG CAG AAC TCT CAG (SEQ ID NO: 2)

Aus dem Plasmid pCM 145 wird nach Verdauung mit den Enzymen Styl und BamHI ein 0,57 kb großes BamHI(4)-Styl(2)-Fragment isoliert (in der publizierten DNA-Sequenz findet sich die Styl-Schnittstelle im Bereich der Aminosäuren 11 bis 13). Dieses Fragment und der vorstehend genannte Linker werden in das mit Ncol und BamHI geschnittene Plasmid pTrc 99 A ligiert, wobei das Plasmid T 297 unter Verlust der Ncol-Schnittstelle erhalten wird.

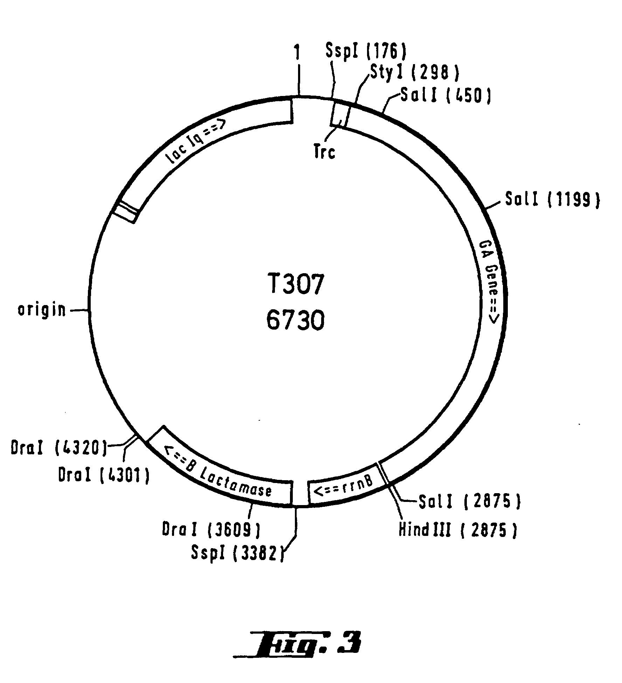

Weiterhin werden aus dem Plasmid pCM 145 ein 1,5 kb Sall-Fragment (Sall(6)-Sall(9)) sowie ein 0,34 kb BamHI-Sall-Fragment isoliert und in den Vektor pUC 18 ligiert, der mit Sall und BamHI geöffnet wurde. Hierbei resuliert das Plasmid T 306, das auf richtige Orientierung des Sall(6-9)-Fragments überprüft wurde. Aus dem Plasmid T 306 wird mit den Enzymen BamHI und HindIII ein 2,1 kb großes Fragment isoliert (das das Fragment ab der BamHI (4)- bis zur Sall(9)-Stelle umfaßt). Dieses Fragment wurde in den mit den Enzymen BamHI und Hindlil geöffneten Vektor T 297 hineinligiert, wobei das Plasmid T 307 (Fig. 3) erhalten wird. Die damit transformierte E. coli Population produziert nach 2-tägiger Fermentation bei 28°C bis 260 U/l GA. Unter geänderten Fermentationsbedingungen kann diese Ausbeute auf über 780 U/l gesteigert werden.

Eine Induktion des Systems mit IPTG bei 37°C ist für den transformierten E. coli-Stamm lethal, was offenbar auf den Einsatz des "high-copy-number-vector" T 307 sowie die gleichzeitig erfolgende Coexpression der ebenfalls sekretierten β-Lactamase zurückzuführen ist. Sie wird daher bei Temperaturen unterhalb von ca. 30°C durchgeführt.

Das Plasmid T 307 weist ein β-Lactamasegen auf, dessen Genprodukt, das Enzym β-Lactamase, zur Selektion rekombinanter Klone dient. Bei der β-Lactamase handelt es sich wie bei der GA um ein sekretiertes, periplasmatisch lokalisiertes Enzym. Eine weitere Verbesserung der Ausbeute konnte durch den Ersatz des β-Lactamasestrukturgens, insbesondere des Signalpeptidbereichs, durch das Chloramphenicol-Acetyltransferasegen erreicht werden. Die Chloramphenicolacetyltransferase findet sich ausschließlich im Cytoplasma der Zelle, wird also nicht in den periplasmatischen Raum ausgeschleust. Die Konstruktionen führten zu dem Plasmid T 363 (Fig. 4), das ebenfalls in der europäischen Anmeldung EP-A-0 504 798 beschrieben ist.

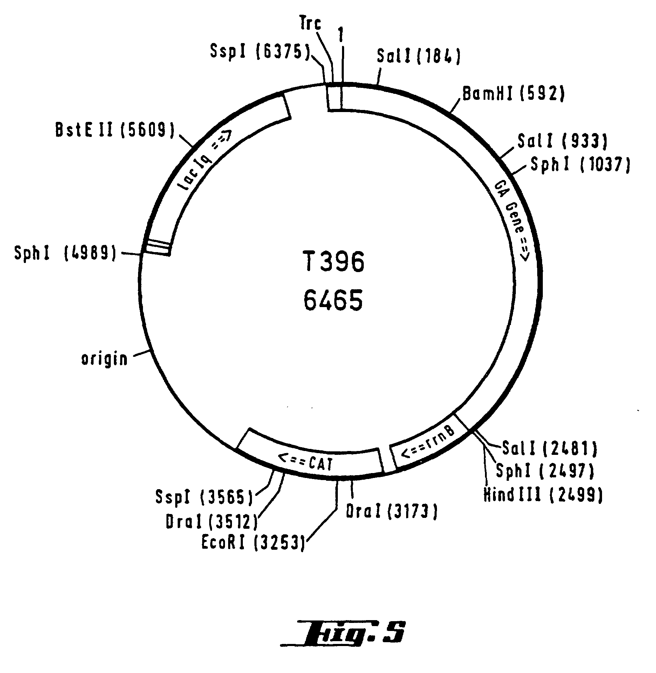

Ein besonderer Vorteil für die Aufarbeitung der GA ergibt sich aus der Verringerung der Expression beispielsweise des CAT-Genprodukts. Exprimiert über seinen natürlichen Promotor im Vektor T 363 ist die Chloramphenicolacetyltransferase ein massiv im Ausschlußmaterial vorkommendes Protein. Durch die Verringerung der Expression verbessert sich das Verhältnis von E.coli-Protein zu GA deutlich zugunsten der GA, wodurch die Trennsäulenbeladung gesteigert und mehr GA pro Zeiteinheit gereinigt werden kann. Die entsprechenden Vektorkonstrukte T 396, T 406 und T 415, die zu einer verringerten CAT-Expression führen, sind in Fig. 5 bis 7 dargestellt.

Die Erfindung bezieht sich daher auch auf ein Verfahren, bei dem das verwendete Selektionsprotein vorzugsweise nur schwach exprimiert wird.

Es wurde weiterhin gefunden, daß die nachstehend erläuterte Optimierung des Fermentationsverfahrens überraschenderweise zu einer Verlängerung der Wachstumsphase der Bakterien, vorzugsweise E. coli, ohne Akkumulation von Acetat führt. Dies führte zusätzlich zu einer Erhöhung der Biomasseausbeuten.

Das Fermentationsverfahren konnte dadurch optimiert werden, daß die Zufütterungsrate der Kohlenstoffquelle während der Hauptkultur, vorzugsweise während der logarhytmischen Wachstumsphase erhöht wird. Als Kohlenstoffquellen können beispielsweise Zucker, Alkohole oder organische Säuren, vorzugsweise Glucose oder Glycerin, insbesondere Glycerin, verwendet werden. Als komplexe Stickstoffquellen können beispielsweise Hefeextrakt, Fleischpeptone, Caseinpeptone und weitere dem Fachmann bekannte komplexe Stickstoffquellen verwendet werden. Die Konzentration der komplexen Stickstoffquellen im Hauptkulturmedium ist ca. 10-50 g/l, insbesondere 20-40 g/l, vorzugsweise ca. 40 g/l.

Die folgenden Beispiele beschreiben nun die zur Erreichung der erhöhten Ausbeuten notwendigen Konstruktionen der Expressionsvektoren sowie die fermentativen Maßnahmen näher. Die für die Klonierung verwendeten Enzyme wurden von New England Biolabs bzw. Gibco/BRL bezogen und entsprechend den Vorschriften der Hersteller verwendet. Alle Angaben hinsichtlich der Größe der Plasmide (bp) sind Richtgrößen.

### Beschreibung der Figuren:

- Fig. 1:: Promotoranschluß des CAT-Gens zur Verringerung der Expressionsrate des CAT-Gens.
Oberer DNA-Strang: SEQ ID NO: 3
Unterer DNA-Strang: SEQ ID NO: 4
1. Leserahmen SEQ ID NO: 5 und SEQ ID NO: 6
2. Leserahmen SEQ ID NO: 7
3. Leserahmen SEQ ID NO: 8 und SEQ ID NO: 9
- Fig. 2-7:: Expressionsvektoren pCM 145, T 307, T 363, T 396, T 406 und T 415.

### Beispiel 1

### Reduktion der Expression des CAT-Gens (Plasmid T 396)

Um die Expression an CAT zu verringern, wurde ein promotorloses CAT-Gen hinter den β-Lactamase Promotor kloniert. Isolierte Plasmid DNA von T 307 (Fig. 3) wurde dazu mit den Restriktionsenzymen Sspl und Dral komplett verdaut und die entstandenen Fragmente auf einem 0,6 % Agarosegel aufgetrennt. Durch Elektroelution wurden dann das ca. 3200 bp große Sstl-Fragment mit dem GA-Gen und das ca. 2600 bp große Sstl-Dral-Fragment mit dem Lac Iq-Gen und dem Replikationsursprung des Vektors isoliert.

Das promotorlose CAT-Gen wurde wie folgt aus dem Plasmid pCM 4 (T. Close und R. Rodriguez, Gene 20 (1982), 305 bis 316) gewonnen. Isolierte Plasmid DNA von pCM 4 wurde mit dem Restriktionsenzym Bam HI komplett geschnitten und die überhängenden Enden mit DNA-Polymere I in Gegenwart von ATP, TTP, CTP und GTP aufgefüllt, so daß Fragmente mit stumpfen Enden entstehen. Das ca. 780 bp große Fragment mit dem CAT-Gen wurde nach Agarose-Gelektrophorese des Verdauungsansatzes in 1,2 % Agarose über Elektroelution isoliert.

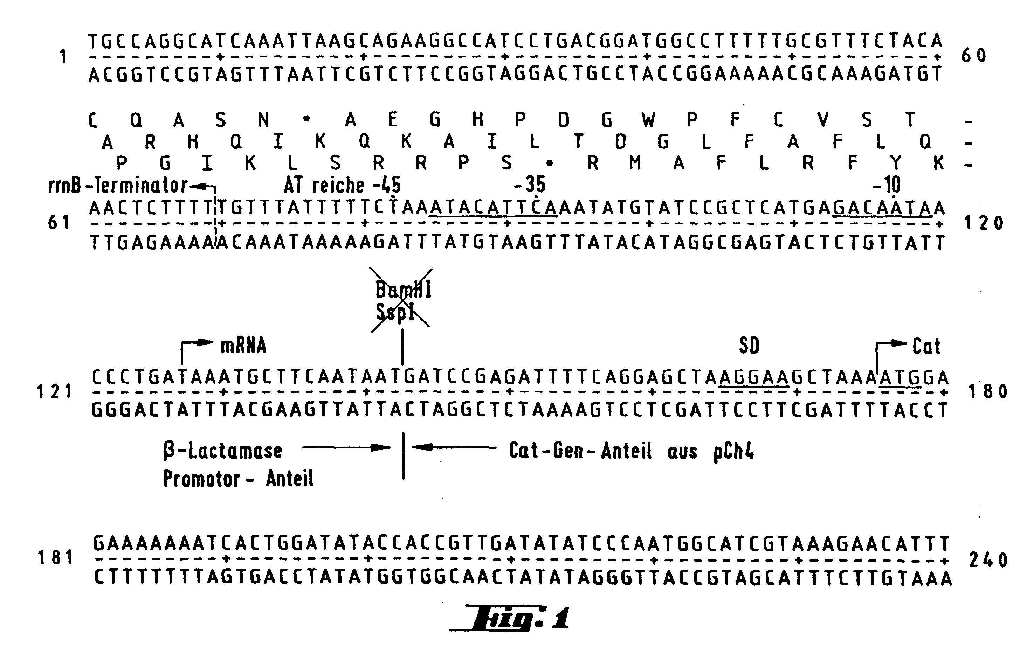

Dieses Fragment sowie die beiden 3200 bp bzw. 2600 bp großen Fragmente aus T 307 wurden dann in einem Ligationsansatz mit Hilfe von DNA-Ligase zusammenligiert. Das Ligationsgemisch wurde in E.coli W 3110 M transformiert und solche rekombinanten Klone isoliert, die nur in Gegenwart verringerter Konzentrationen an Chloramphenicol (12,5 µg/ml) optimal wuchsen und Glutarylamidasebildung gezeigten. Isolierte und Chloramphenicol-resistente Klone wiesen Plasmide mit den drei Fragmenten auf, wobei das 2600 bp Fragment in 2 Orientierungen vorlag. Beste Produktausbeute ergab sich mit Hilfe des Plasmids T 396. Durch die nicht optimale Anpassung des CAT-Gens an den β-Lactamase Promotor ergibt sich der geringere Resistenzlevel im Vergleich zu T 363 (25 µg/ml). Der Anschluß des CAT-Gens an dem verbleibenden Rest der β-Lactamasepromotorsequenz ist in Fig. 1 dargestellt.

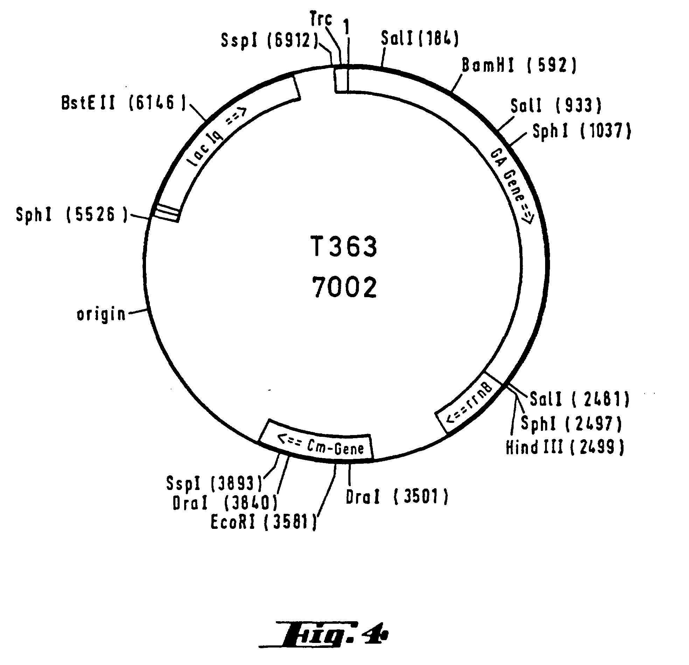

Die Analyse der von dem E.coli Stamm W 3110 (T 396) gebildeten Proteine im 10 bis 17,5 % SDS-Polyacrylamidgel nach 2 tägigem Wachstum der Zellen unter Induktionsbedingungen belegt im Vergleich zu den Proteinbanden des unter gleichen Bedingungen angezüchteten Stammes W 3110 (T 363; Fig. 4) eine Verminderung des CAT-Proteins um mehr als 80 %. Bei annähernd ähnlichen Ausbeuten an GA-Enzym in beiden Stämmen verbessert sich somit das Mengenverhältnis nach Aufschluß der Zellen von gelöst vorliegender GA zu löslichem Gesamtprotein signifikant, was wegen der geringeren Menge an Fremdprotein in Bezug zur GA zu einer deutlich vereinfachten Aufarbeitung der Glutarylamidase führt.

### Beispiel 2:

### Inaktivierung des Lac I^{q}-Repressor Gens (Plasmid T 415)

Schüttelkulturversuche und Fermentationen hatten ergeben, daß bei allen für die GA-Bildung verwendeten Plasmiden und E.coli Stämmen die GA-Synthese vor Induktion mit IPTG nicht, wie erwartet, weitgehend abgeschaltet war. Auch entsprachen die für das IPTG abhängige Lac-Induktionssystem beschriebenen Induktionsraten nicht den Literaturwerten. Um die GA-Expression komplett unabhängig zu machen von dem Gebrauch eines Induktors wie IPTG, wurde der für den Lac I^{q}-Repressor kodierende Leserahmen zerstört. Ein inaktiver Repressor resultiert, wodurch die Blockierung der Ablesung des Gens in Abwesenheit von Induktor aufgehoben ist.

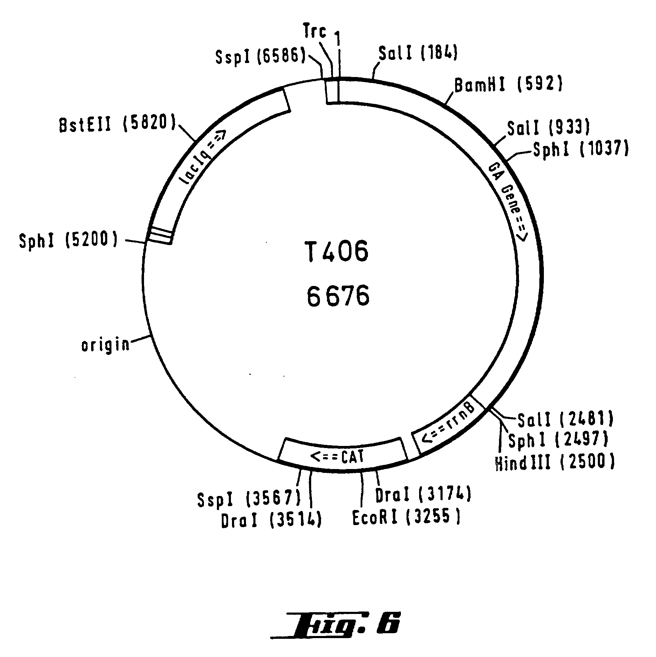

Isolierte Plasmid DNA von T 396 (Fig. 5) wurde mit den Restriktionsenzymen EcoRI und HindIII geschnitten und das ca. 750 bp Fragment mit dem Anschlußteil des CAT-Gens mit dem β-Lactamase Promotor, der zur verringerten CAT-Genexpression führte, durch Elektroelution isoliert. Dieses Insert wurde dann mit dem ebenfalls isolierten ca. 6000 bp großen EcoRl-Hindlll Fragment aus dem Plasmid T 363 zusammenligiert. Es resultiert der Expressionsvektor T 406 (Fig. 6). In der Fermentation hat es sich nun überraschenderweise gezeigt, daß der Vector T 406 stabiler ist als T 396.

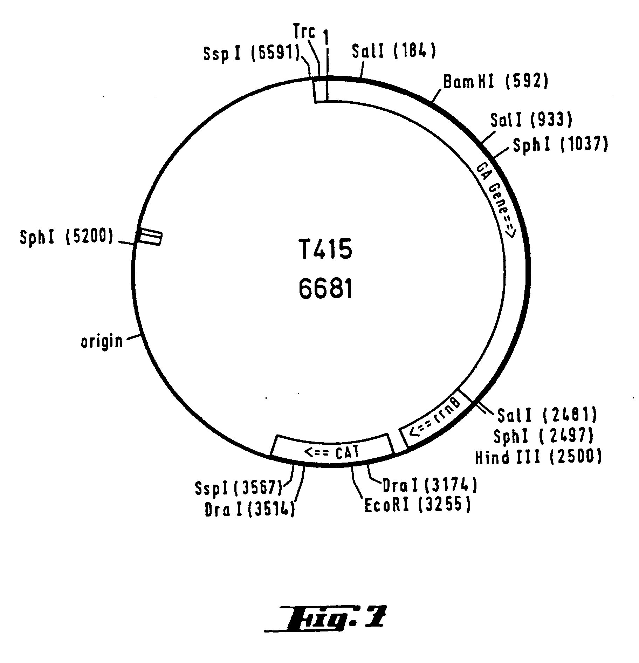

Zur Inaktivierung des lac I^{q} Gens wurde isolierte Plasmid DNA von T 406 mit dem Restriktionsenzym BstEII komplett verdaut. Die singuläre Schnittstelle in dem Vektor liegt im Strukturgen für den Repressor. Die überhängenden Enden wurden in Gegenwart von d ATP, d TTP, d GTP und d CTP mit mittels DNA-Polymerase aufgefüllt und der Vektor in Gegenwart von DNA-Ligase religiert. Durch diesen Schritt erfolgt ein Leserahmenwechsel um 2 Basenpaare, so daß kein funktionsfähiger Lac I^{q}-Repressor gebildet werden kann. Es resultiert das Plasmid T 415 (Fig. 7). Rekombinante E.coli Klone W 3110 (T 415) zeigen in der Schüttelkultur einen Anstieg der Bildung von GA von ca. 510 U/l nach 2 Tagen (T 363 mit Induktor) auf 1590 U/l bei T 415, ohne daß Induktor zugegeben wurde. Durch Zugabe von Induktor zum T 415-System wird die Ausbeute in einem Kontrollexperiment nicht verbessert.

Die für E.coli Stämme mit dem Vektor T 396 gezeigten Aufarbeitungsvorteile gelten in gleichem Maße für E.coli mit dem Plasmid T 415.

Da die Inaktivierung des Lac I^{q}-Repressorgens sich als vorteilhaft erwiesen hat, ist es auch denkbar, in E.coli replizierende Vektoren für die Konstruktionen zur konstitutiven Expression der GA zu benutzen, in denen das Lac I^{q} Gen von vornherein fehlt. Ebenso erscheint es möglich, E.coli Stämme für die Produktion der GA zu verwenden, denen bei Verwendung des Lac Promotor-Operator Systems die Expression des Lac-Wildtyp Repressors bzw. der Mutanten davon fehlt oder in geringem Maße erfolgt.

### Beispiel 3:

### Fermentation von E.coli T 415

Im Gegensatz zu den E.coli-Klonen T 347 und T 362/33 gemäß EP-A- 0 504 798 braucht bei dem Konstrukt T 415 die Glutaryl-Amidase (GA) nicht mehr durch IPTG induziert zu werden. Die optimierte Fermentation wird wie folgt beschrieben:

### Anzuchtbedingungen:

Alle Anzuchten wurden, mit Ausnahme der zu variierenden Parameter, unter folgenden Bedingungen durchgeführt:

Die Stammhaltung erfolgte vorzugsweise bei -18°C im YT-Glycerin-Medium:

| | |
|---|---|
| Glycerin | 17,0 % |
| Hefeextrakt | 0,7 % |
| Bactotryptone | 0,4 % |
| NaCl | 0,4 % |
| Chloramphenicol | 12,5 µg/ml |

Aus dieser Suspension wurden Agarplatten des gleichen Mediums angelegt, 24 Std. bei 28°C inkubiert und die Vorkultur (VK) mit einer Einzelkolonie inokuliert.

| | | |
|---|---|---|
| VK-Medium (NL 5295) | Bacto-Trypton | 2,0 % |
| | Bacto-Hefeextrakt | 1,0 % |
| | NaCl | 0,5 % |
| | Chloramphenicol | 12,5 µg/ml |
| pH = 7,2 | | |

100 ml dieser Nährlösung in 300 ml Erlenmeyerkolben wurden nach dem Animpfen für 16 bis 24 Std. bei 28°C und 220 Upm inkubiert. Die Kultur zeigte dann eine OD₅₇₈ₙₘ von 6,0 bis 8,0.

Aus dieser VK wurde die folgende Hauptkultur (HK) mit 5 bis 10 % (bezogen auf VK mit OD₅₇₈ₙₘ = 3,0) beimpft:
HK: NL 5292 + 1 ml Desmophen

| | |
|---|---|
| 20,0 g/l | Hefextrakt (Oxoid) |
| 1,2 g/l | NaH₂PO₄ x H₂O |
| 8,5 g/l | Na₂HPO₄ x 2H₂O |
| 1,0 g/l | Kcl |
| 2,0 g/l | MgSO₄ x 7H₂O (separat autoklaviert) |
| 0,25 g/l | Zitronensäure |
| 5,0 g/l | NH₄Cl |
| 4,0 g/l | SLA 5029 |
| 0,005 g/l | Thiamin → sterilfiltriert (5 mg/10 ml → 0,5/50 ml NL) |
| pH = 6,5 | |

| | | |
|---|---|---|
| Ferm.beding. | Temp. | 28°C |
| | Vol. | 3,5 L |
| | vvm | 0,75 |
| | Upm | 500 (r = 7 cm) |
| | pH | 7,0 ± 0,2 (mit NH₄OH 25 %ig konstanthalten) |

| | | |
|---|---|---|
| Fedbatch | Glycerinlösung | 525 g Glycerin (99 %)/l HK-Medium (ohne NH₄Cl) |
| | Fütterungsrate | 3,4 ml/L*Std. bei kontinuierlicher Zugabe (max. Glycerinkonz. im Fermenter: 0,2 %) |
| | Fütterungsbeginn | in der 5. Fermentationsstunde |
| | Fütterungsdauer | 40 bis 70 Std. |
| | pO₂ | bei ca. 40 % konstanthalten |

### Beispiel 4: Nachweis der IPTG-Unabhängigkeit der GA-Expression

Zu diesem Zweck wurde der Klon T 415 unter obigen Bedingungen im Fermenter angezogen und zu verschiedenen Zeitpunkten jeweils zwei Erlenmeyerkolben mit 50 ml Kulturlösung abgefüllt. Ein Kolben wurde mit 1 mM IPTG induziert, der zweite nicht. Folgende GA-Volumenaktivitäten wurden gemessen:

| Abnahmezeitpunkt | GA (U/L)* | |
|---|---|---|
| | +IPTG | ohne IPTG |
| 23. Std. | 2700 | 2800 |
| 31. Std. | 2600 | 2600 |
| 47. Std. | 4400 | 4500 |

| | | |
|---|---|---|
| *24 Std. nach Kolbenabfüllung | | |

Der Versuch macht deutlich, daß IPTG zu GA-Induktion nicht mehr notwendig ist.

### Beispiel 5: Fermentation von E.coli T 415

Unter oben beschriebenen Fermentationsbedingungen wurden nach 73 Stunden 8200 U GA/L Kulturlösung (KL) erreicht. Die spezifische Aktivität betrug ca. 70 U/g Feuchtmasse. Biomasseausbeute ca. 120 g Feuchtmasse/ L.

### Beispiel 6: Fermentation mit modifiziertem HK-Medium

Eine Verdopplung der Hefeextraktkonzentration im HK-Medium von 20 auf 40 g/L führte zu einer Steigerung der Wachstumsgeschwindigkeit und einer Erhöhung der Biomasseausbeute auf ca. 140 g/ L. Die GA-Volumenaktivität stieg auf 9600 U/ L Kl nach 73 Stunden. 8000 U/ L wurden bereits nach 48 Stunden erreicht, was eine Verkürzung der Fermentationsdauer um einen Tag bedeutet.

### Beispiel 7: Fermentation mit modifizierter Fedbatch-Fütterung

Durch die Eliminierung der IPTG-abhängigen Regulation der GA-Expression konnte nicht nur eine erfolgreiche Aufstockung des HK-Mediums ausprobiert werden (Beispiel 6), sondern auch die bis dahin limitierende Glycerin-Zufütterungsrate bedingt durch Acetalbildung variiert werden. Erhöhte man beispielsweise die Zufütterungszurate von der 5. bis zu 8. Fermentationsstunde kontinuierlich von 3,4 m)/L*Std. auf 6,6 mi/L*Std. und hält sie dann bis Fermentationsende konstant, so führte dies zu einer Verlängerung der schnellen, optimalen Wachstumsphase mit den Resultat, daß der Klon bis zur 73. Stunde konstant das Enzym mit gesteigerter Syntheserate bildete und letztlich bis zu 18800 U/L KL produziert wurden. Unter Berücksichtigung der spezifischen Aktivität der GA von 7 U/mg Protein wurden somit über 2,5 g Enzym pro Liter KL gebildet. Die Biomasseausbeute betrug ca. 200 g/L KL. Intermediär kam es nur noch zu geringer Acetatbildung von max. 3-4 g/L.

## Patentansprüche

1. Gentechnisches Verfahren zur Herstellung von Glutarylacylase (GA) in Bakterien, **dadurch gekennzeichnet, dass** das GA-Gen konstitutiv von einem Expressionsvektor exprimiert wird unter Verwendung eines Lac-Promotor-Operator-Systems, bei dem das Lac I^{q}-Repressorgen fehlt oder inaktiviert ist.

2. Verfahren nach Anspruch 1, **dadurch gekennzeichnet, dass** der Promotor zur konstitutiven Expression des GA-Gens ein Trc- oder Trc-äquivalenter Promotor ist, der keinen oder einen nicht-funktionellen Repressor besitzt.

3. Verfahren nach Anspruch 1 oder 2, **dadurch gekennzeichnet, dass** der genannte Expressionsvektor zusätzlich ein Selektionsgen enthält, das für ein in Bakterien bleibendes, nicht periplasmatisches Protein kodiert.

4. Verfahren nach Anspruch 3, **dadurch gekennzeichnet, dass** die Expression des genannten Proteins gegenüber einer Expression mit dessen natürlichem Promotor verringert ist.

5. Verfahren nach Anspruch 3 oder 4, **dadurch gekennzeichnet, dass** das Selektionsgen für Chloramphenicolacyltransferase (CAT) kodiert.

6. Verfahren nach einem der Ansprüche 1-5, **dadurch gekennzeichnet, dass** bei der Fermentation der Bakterien die Zufütterungsrate der Kohlenstoffquelle während der Hauptkultur erhöht wird.

7. Verfahren nach Anspruch 6, **dadurch gekennzeichnet, dass** die genannte Zufütterungsrate während der logarhythmischen Wachstumsphase erhöht wird.

8. Verfahren nach Anspruch 6 oder 7, **dadurch gekennzeichnet, dass** die genannte Kohlenstoffquelle Glycerin ist.

9. Verfahren nach einem der Ansprüche 1 - 8, **dadurch gekennzeichnet, dass** bei der Fermentation der Bakterien ca. 10-50 g/l einer komplexen Stickstoffquelle verwendet wird.

10. Verfahren nach einem der Ansprüche 1 - 9, **dadurch gekennzeichnet, dass** es sich bei den Bakterien um E. coli handelt.

## Claims

1. Genetical process for the preparation of glutarylacylase (GA) in bacteria, **characterised in that** the GA gene is constitutively expressed from an expression vector using a Lac-promoter-operator system, in which the Lac I^{q} repressor gene is missing or has been inactivated.

2. Process according to claim 1, **characterised in that** the promoter for constitutive expression of the GA gene is a Trc or Trc-equivalent promoter, which has no or a non-functional repressor.

3. Process according to claim 1 or 2, **characterised in that** the said expression vector additionally contains a selection gene, which codes for a non-periplasmatic protein remaining in bacteria.

4. Process according to claim 3, **characterised in that** expression of the said protein is diminished compared with expression with its natural promoter.

5. Process according to claim 3 or 4, **characterised in that** the selection gene codes for chloramphenicol acyltransferase (CAT).

6. Process according to one of claims 1-5, **characterised in that** during fermentation of the bacteria, the feed rate for the source of carbon is increased during the main culture.

7. Process according to claim 6, **characterised in that** the said feed rate is increased during the logarithmic growth phase.

8. Process according to claim 6 or 7, **characterised in that** the said source of carbon is glycerol.

9. Process according to one of claims 1-8, **characterised in that** during fermentation of the bacteria, ca. 10 - 50 g/1 of a complex source of nitrogen is used.

10. Process according to one of claims 1-9, **characterised in that** the bacteria in question is *E. coli.*

## Revendications

1. Procédé de génie génétique pour la préparation de la glutarylacylase (GA) dans des bactéries, **caractérisé en ce que** le gène-GA constitutif d'un vecteur d'expression est exprimé en utilisant un système opérateur-promoteur-lac, dans lequel le gène répresseur Lac I^{q} est absent ou est inactivé.

2. Procédé selon la revendication 1, **caractérisé en ce que** le promoteur pour l'expression constitutive du gène-GA est un promoteur Trc- ou Trc-équivalent, qui ne comporte pas de répresseur ou en comporte un qui est non fonctionnel.

3. Procédé selon la revendication 1 ou 2, **caractérisé en ce que** le vecteur d'expression mentionné comporte en outre un gène de sélection, qui code pour une protéine non périplasmatique restant dans les bactéries.

4. Procédé selon la revendication 3, **caractérisé en ce que** l'expression de la protéine citée est diminuée par rapport à une expression avec son promoteur naturel.

5. Procédé selon la revendication 3 ou 4, **caractérisé en ce que** le gène de sélection code pour la chloramphénicolacyltransférase (CAT).

6. Procédé selon l'une des revendications 1 à 5, **caractérisé en ce que** pour la fermentation des bactéries, la vitesse d'alimentation de la source de carbone est augmentée pendant la culture principale.

7. Procédé selon la revendication 6, **caractérisé en ce que** la vitesse d'alimentation mentionnée est augmentée pendant la phase de croissance logarithmique.

8. Procédé selon la revendication 6 ou 7, **caractérisé en ce que** la source de carbone mentionnée est la glycérine.

9. Procédé selon l'une des revendications 1 à 8, **caractérisé en ce que**, pour la fermentation des bactéries, on utilise 10-50 g/l d'une source d'azote complexe.

10. Procédé selon l'une des revendications 1 à 9, **caractérisé en ce que** les bactéries sont E. coli.
